# EUROPEAN PATENT SPECIFICATION

(11) **EP 4 475 870 B1**
(45) Date of publication and mention of the grant of the patent: **24.06.2026**
(21) Application number: 23704926.7
(22) Date of filing: 10.02.2023
(51) Int. Cl.: A61K 38/17, A61P 7/02

(54) **SOLUBLE ENDOGLIN FOR USE IN TREATING OR PREVENTING THROMBUS FORMATION**
SOLUBILES ENDOGLIN ZUR BEHANDLUNG ODER VERHINDERUNG DER THROMBUSBILDUNG
ENDOGLINE SOLUBLE POUR LE TRAITEMENT OU LA PRÉVENTION DE LA FORMATION DE THROMBUS

(30) Priority: 11.02.2022 EP 22305156
(43) Date of publication of application: 18.12.2024
(73) Proprietor: Institut National de la Santé et de la Recherche Médicale (INSERM), 75013 Paris (FR); Université Paris Cité, 75006 Paris (FR)
(72) Inventor: ROSSI, Elisa, 75006 Paris (FR)
(74) Representative: Inserm Transfert
(86) International application number: PCT/EP2023/053305
(87) International publication number: WO 2023/152291

(56) References cited:
- WO-A1-2007/102562
- WO-A1-2013/019805
- WO-A1-2016/030316
- WO-A1-2018/086540
- CN-A- 110 857 318
- US-A1- 2003 199 457
- PERICACHO ET AL.: "Impaired hemostasis in HHT animal models due to alterationsin thrombus stabilization12th International HHT Scientific Conference ED - Nowak-Sliwinska Patrycja; Griffioen Arjan W", ANGIOGENESIS, KLUWER, DORDRECHT, NL, vol. 21, no. 1, 20 November 2017 (2017-11-20), pages 111 - 167, XP036430296, ISSN: 0969-6970, [retrieved on 20171120], DOI: 10.1007/S10456-017-9584-3
- ROSSI ELISA ET AL: "Human endoglin as a potential new partner involved in platelet-endothelium interactions", CMLS CELLULAR AND MOLECULAR LIFE SCIENCES, BIRKHAUSER VERLAG, HEIDELBERG, DE, vol. 75, no. 7, 28 October 2017 (2017-10-28), pages 1269 - 1284, XP036451477, ISSN: 1420-682X, [retrieved on 20171028], DOI: 10.1007/S00018-017-2694-7

## Description

### FIELD:

The present disclosure relates to methods and pharmaceutical composition for treating thrombotic disorders.

### BACKGROUND:

Endoglin (Eng) is a membrane co-receptor for the transforming growth factor-beta (TGF-β) family that is overexpressed on proliferating endothelial cells and involved in different cardiovascular conditions¹. Mutations in the endoglin gene (ENG) cause hereditary hemorrhagic telangiectasia (HHT) type¹. HHT is a disease characterized by the presence of arteriovenous malformations (AVMs) in the brain, liver and lung, as well as mucocutaneous telangiectases leading to epistaxis and gastrointestinal (GI) bleeding^{2,3}. Spontaneous and recurrent epistaxis is the hallmark of HHT and is the most common clinical manifestation affecting over 90% of patients, some of whom presenting with multiple bleeds per day⁴. Moreover, GI bleeding affects up to 25% of HHT patients. Chronic nasal and GI bleeding can cause iron-deficiency anemia and there is an active search for therapeutic strategies to minimize iron deficiency and blood transfusions and to alleviate symptoms. It is widely admitted that bleeding in HHT is mainly due to the rupture of the fragile nasal and GI telangiectasias, although the additional involvement of an abnormal hemostasis cannot been excluded, especially in the HHT1 variant generated by mutations in ENG. In this line, we previously showed that the extracellular region of membraine Eng has a role in hemostasis via the αIIbβ3 integrin-mediated adhesion of platelets to the endothelium⁵. αIIbβ3, also named glycoprotein GPIIb-IIIa (CD41/CD61) complex, is the most numerous integrin found on platelets. Each platelet expresses around 80,000 copies of αIIbβ3 on its surface, and can mobilize an additional pool of around 30,000 receptors upon platelet activation, secondary to secretion and exposure of α-granule membranes at the plasma surface. αIIbβ3 is crucial in hemostasis due to its central role in supporting stable platelet adhesion and aggregation⁶. Upon the proteolytic processing of membrane-bound Eng, catalyzed by the metalloprotease matrix metalloproteinase-14 (MMP14) or -12 (MMP12), a circulating form (soluble endoglin, sEng), encompassing the extracellular region of Eng, can be released⁷⁻⁹. Interestingly, sEng has been found to counteract the function of membrane-bound endoglin, by competing with its binding to integrin α5β1^{10,11} and αIIbβ3^{5,12}. Circulating sEng is found at low concentrations in the plasma of healthy subjects, but its levels are increased in various pathological conditions related to the endothelium, as reported in preeclampsia^{13,14}, atherosclerosis, hypercholesterolemia¹⁵, diabetes mellitus¹⁶, hypertension¹⁷, coronary artery disease¹⁸ and acute myocardial infarction¹⁹. It has been postulated that the upregulated levels of sEng in plasma, serum or urine of certain patients are a reliable marker of severity and prognosis of these diseases. Many of these pathological conditions also involve the hemostasis system driven by platelets. However, the role of sEng in platelet-dependent hemostasis is unknown. Conversely, downregulated levels of plasma sEng have been reported in patients with HHT (Ojeda-Fernandez et al., 2010; Wetzel-Strong et al., 2021). Interestingly, despite their bleeding propensity, HHT patients also suffer from thrombotic complications, including paradoxical thromboembolic stroke from pulmonary AVMs, deep vein thrombosis and abnormal endothelium-dependent hemostasis (Faughnan et al., 2011; Dittus et al., 2015; Chaturvedi et al., 2018; Riera-Mestre et al., 2019). Accordingly, treatment strategies in HHT include antithrombotic therapy (Gaetani et al. 2020). However, the precise indications and safety of antithrombotic therapy in HHT patients, as well as the molecular mechanisms involved are lacking. WO2013019805 discloses the use of soluble endoglin in the treatment of a cardiovascular condition such as heart failure (HF) and myocardial infarction (MI) and other cardiovascular diseases.

Due to the high bleeding risk associated with the use of molecules such as eptifibatide and abciximab, their use is currently limited to acute settings such as during percutaneous coronary intervention (PCI) or acute coronary syndrome (ACS) in patients with high thrombotic risk. The development of oral active agents was halted due to an unexpected 30-35% increase in mortality, which is paradoxically linked to the ability of these drugs to activate platelet integrins²⁵. Therefore, there is a current need to find alternative and safer therapeutic approaches. Arterial thrombosis (blood clot) and emboli formation are a common complication of many systemic diseases associated with chronic inflammation. Since thrombi result from inappropriate platelet activation and subsequent coagulation, therapeutic targeting of these systems has important clinical significance for developing safer treatments.

Herein the inventors demonstrate the antithrombotic properties of sEng, suggesting that sEng may be a novel therapeutic tool/target in hemostasis, to decrease or dissolve clots.

### SUMMARY OF THE INVENTION:

In a first aspect, the present invention relates to a soluble endoglin for use for reducing or preventing thrombus formation in a subject in need thereof.

In particular, the present invention is defined by the claims.

### DETAILED DESCRIPTION:

Under flow conditions, the inventors demonstrate that supplementation of human whole blood with sEng led to a slower thrombus formation and a smaller thrombus size than controls. Moreover they show that sEng inhibited platelet aggregation and thrombus retraction. Molecular modelling showed a good fitting between αIIbβ3 and sEng structures involving the endoglin RGD motif, making the αIIbβ3/sEng complex highly stable. By competition assays with fibrinogen, sEng reduces platelets aggregation.

These results show that sEng interferes with thrombus formation and stabilization likely via its binding to platelet αIIbβ3, suggesting that sEng could be a new tool for antithrombotic therapy.

### Methods of the invention

Accordingly, the invention refers to a method for reducing or preventing thrombus formation in a subject in need thereof comprising administering to the subject a therapeutically effective amount of a soluble endoglin.

The method of the present invention is thus particularly suitable for the treatment of thrombotic disorders. Thus, the invention refers to a method for reducing or preventing thrombus formation in a subject in need thereof comprising administering to the subject a therapeutically effective amount of a soluble endoglin, wherein the thrombus is caused by thrombotic disorders.

In other words, the invention refers to a soluble endoglin for use as antithrombotic agent.

In other words, the invention refers to a soluble endoglin for use as a medicament for reducing or preventing thrombus formation in a subject in need thereof.

As used herein, the term "soluble endoglin" , also known as "sEng", has its general meaning in the art and refers to the circulating form of endoglin released by the proteolytic processing of endoglin by the metalloprotease matrix metalloproteinase-14 (MMP14) or -12 (MMP12) ⁷⁻⁹. Soluble endoglin encompasses thus the extracellular region of endoglin.

In particular embodiment, the soluble endoglin comprises or refers to the amino acid sequence of SEQ ID NO: 1 (which corresponds to human endoglin soluble (i.e peptide signal signal and the extracellular domain human endoglin amino acid sequence)), or SEQ ID NO:2 (which correspond to the human endoglin soluble sequence without its peptide signal sequence) and any natural variant thereof (e.g., variants present in other animal species, or variants as a result of polymorphism or splicing). Within the context of the present invention, the term "soluble endoglin" also includes any polypeptide comprising a sequence having at least 90% sequence identity to the sequence shown in SEQ ID NO: 1 or in SEQ ID NO:2.

In preferred embodiment, the soluble endoglin comprises or refers to SEQ ID NO:1

In some embodiment, the soluble endoglin is a dimeric soluble endoglin, i.e comprises two amino acid sequence set forth as SEQ ID NO: 1 or SEQ ID NO:2.
SEQ ID NO:1 >sp|P17813|soluble endoglin 
MDRGTLPLAVALLLASCSLSPTSLAETVHCDLQPVGPERGEVTYTTSQVSKGCVAQAPNAILEVHVL
FLEFPTGPSQLELTLQASKQNGTWPREVLLVLSVNSSVFLHLQALGIPLHLAYNSSLVTFQEPPGVN
TTELPSFPKTQILEWAAERGPITSAAELNDPQSILLRLGQAQGSLSFCMLEASQDMGRTLEWRPRTP
ALVRGCHLEGVAGHKEAHILRVLPGHSAGPRTVTVKVELSCAPGDLDAVLILQGPPYVSWLIDANHN
MQIWTTGEYSFKIFPEKNIRGFKLPDTPQGLLGEARMLNASIVASFVELPLASIVSLHASSCGGRLQ
TSPAPIQTTPPKDTCSPELLMSLIQTKCADDAMTLVLKKELVAHLKCTITGLTFWDPSCEAEDRGDK
FVLRSAYSSCGMQVSASMISNEAVVNILSSSSPQRKKVHCLNMDSLSFQLGLYLSPHFLQASNTIEP
GQQSFVQVRVSPSVSEFLLQLDSCHLDLGPEGGTVELIQGRAAKGNCVSLLSPSPEGDPRFSFLLHF
YTVPIPKTGTLSCTVALRPKTGSQDQEVHRTVFMRLNIISPDLSGCTSKG
SEQ ID NO:2 >sp|P17813|soluble endoglin (without peptide signal)

ETVHCDLQPVGPERGEVTYTTSQVSKGCVAQAPNAILEVHVLFLEFPTGPSQLELTLQASKQNGTWP
REVLLVLSVNSSVFLHLQALGIPLHLAYNSSLVTFQEPPGVNTTELPSFPKTQILEWAAERGPITSA
AELNDPQSILLRLGQAQGSLSFCMLEASQDMGRTLEWRPRTPALVRGCHLEGVAGHKEAHILRVLPG
HSAGPRTVTVKVELSCAPGDLDAVLILQGPPYVSWLIDANHNMQIWTTGEYSFKIFPEKNIRGFKLP
DTPQGLLGEARMLNASIVASFVELPLASIVSLHASSCGGRLQTSPAPIQTTPPKDTCSPELLMSLIQ
TKCADDAMTLVLKKELVAHLKCTITGLTFWDPSCEAEDRGDKFVLRSAYSSCGMQVSASMISNEAVV
NILSSSSPQRKKVHCLNMDSLSFQLGLYLSPHFLQASNTIEPGQQSFVQVRVSPSVSEFLLQLDSCH
LDLGPEGGTVELIQGRAAKGNCVSLLSPSPEGDPRFSFLLHFYTVPIPKTGTLSCTVALRPKTGSQD
QEVHRTVFMRLNIISPDLSGCTSKG

The soluble endoglin of the invention may be produced by any suitable means, as will be apparent to those of skill in the art. **In** order to produce sufficient amounts of soluble endoglin or functional equivalents thereof for use in accordance with the present invention, expression may conveniently be achieved by culturing under appropriate conditions recombinant host cells containing the soluble endoglin of the invention. **In** particular, the endoglin soluble is produced by recombinant means, by expression from an encoding nucleic acid molecule. Systems for cloning and expression of a polypeptide in a variety of different host cells are well known. When expressed in recombinant form, the polypeptide is in particular generated by expression from an encoding nucleic acid in a host cell. Any host cell may be used, depending upon the individual requirements of a particular system. Suitable host cells include bacteria mammalian cells, plant cells, yeast and baculovirus systems. Mammalian cell lines available in the art for expression of a heterologous polypeptide include Chinese hamster ovary cells. HeLa cells, baby hamster kidney cells and many others. Bacteria are also preferred hosts for the production of recombinant protein, due to the ease with which bacteria may be manipulated and grown. A common, preferred bacterial host is E coli. In particular, the endoglin soluble of the invention is produced by transfecting a vector encoding the human endoglin soluble in Chinese hamster ovary cells, such as CHO-K1 cells, as described in Ruiz-Llorente et al. IJMS, 202.

The endoglin soluble of the invention can exhibit post-translational modifications, including, but not limited to glycosylations, (e.g., N-linked or O-linked glycosylations), myristylations, palmitylations, acetylations and phosphorylations (e.g., serine/threonine or tyrosine).

In specific embodiments, it is contemplated that soluble endoglin used in the therapeutic methods of the present invention may be modified in order to improve their therapeutic efficacy. Such modification of therapeutic compounds may be used to decrease toxicity, increase circulatory time, or modify biodistribution. For example, the toxicity of potentially important therapeutic compounds can be decreased significantly by combination with a variety of drug carrier vehicles that modify biodistribution.

A strategy for improving drug viability is the utilization of water-soluble polymers. Various water-soluble polymers have been shown to modify biodistribution, improve the mode of cellular uptake, change the permeability through physiological barriers; and modify the rate of clearance from the body. To achieve either a targeting or sustained-release effect, water-soluble polymers have been synthesized that contain drug moieties as terminal groups, as part of the backbone, or as pendent groups on the polymer chain.

Polyethylene glycol (PEG) has been widely used as a drug carrier, given its high degree of biocompatibility and ease of modification. Attachment to various drugs, proteins, and liposomes has been shown to improve residence time and decrease toxicity. PEG can be coupled to active agents through the hydroxyl groups at the ends of the chain and via other chemical methods; however, PEG itself is limited to at most two active agents per molecule. In a different approach, copolymers of PEG and amino acids were explored as novel biomaterials which would retain the biocompatibility properties of PEG, but which would have the added advantage of numerous attachment points per molecule (providing greater drug loading), and which could be synthetically designed to suit a variety of applications.

Those of skill in the art are aware of PEGylation techniques for the effective modification of drugs. For example, drug delivery polymers that consist of alternating polymers of PEG and tri-functional monomers such as lysine have been used by VectraMed (Plainsboro, N.J.). The PEG chains (typically 2000 daltons or less) are linked to the a- and e-amino groups of lysine through stable urethane linkages. Such copolymers retain the desirable properties of PEG, while providing reactive pendent groups (the carboxylic acid groups of lysine) at strictly controlled and predetermined intervals along the polymer chain. The reactive pendent groups can be used for derivatization, cross-linking, or conjugation with other molecules. These polymers are useful in producing stable, long-circulating pro-drugs by varying the molecular weight of the polymer, the molecular weight of the PEG segments, and the cleavable linkage between the drug and the polymer. The molecular weight of the PEG segments affects the spacing of the drug/linking group complex and the amount of drug per molecular weight of conjugate (smaller PEG segments provides greater drug loading). In general, increasing the overall molecular weight of the block co-polymer conjugate will increase the circulatory half-life of the conjugate. Nevertheless, the conjugate must either be readily degradable or have a molecular weight below the threshold-limiting glomular filtration (e.g., less than 60 kDa).

In addition, to the polymer backbone being important in maintaining circulatory half-life, and biodistribution, linkers may be used to maintain the therapeutic agent in a pro-drug form until released from the backbone polymer by a specific trigger, typically enzyme activity in the targeted tissue. For example, this type of tissue activated drug delivery is particularly useful where delivery to a specific site of biodistribution is required and the therapeutic agent is released at or near the site of pathology. Linking group libraries for use in activated drug delivery are known to those of skill in the art and may be based on enzyme kinetics, prevalence of active enzyme, and cleavage specificity of the selected disease-specific enzymes. Such linkers may be used in modifying the polypeptide or fragment of the polypeptide described herein for therapeutic delivery.

As used herein, the term "subject" denotes a mammal, such as a rodent, a feline, a canine, and a primate. Preferably, a subject according to the invention is a human. Preferably, the subject according to the invention is a human afflicted with or susceptible to be afflicted with thrombotic disorders. In some embodiment, the subject is a human afflicted with or susceptible to be afflicted with hereditary hemorrhagic telangiectasia (HHT), embolism (pulmonary and cerebral), COVID-19 infection and cardiovascular disease such as stroke.

As used herein, the term "antithrombotic agent" has its general meaning in the art and refers to compounds, drugs, therapy used to prevent and/or treat thrombosis in subject. Antithrombotic agent includes anticoagulants agent (which inhibit various aspects of the coagulation pathways) and the antiplatelet agents (which inhibit platelet function).

In particular embodiment, the thrombus are caused by a thrombotic disorders.

As used herein, the term "thrombotic disorders", also known as clotting disorders or thrombophilia, has its general meaning in the art and refers to an inherited or acquired condition that increases the risk of excessive blood clot formation. When a blood vessel is injured, it begins to leak blood either externally or into the tissues. Normal coagulation is important during an injury, as it helps stop a cut from bleeding and starts the healing process. However, if blood tends to clot too much, it is referred to as a hypercoagulable state or thrombophilia. In healthy people, homeostatic balance exists between procoagulant (clotting) forces and anticoagulant and fibrinolytic forces. Numerous genetic, acquired, and environmental factors can tip the balance in favor of coagulation, leading to the pathologic formation of thrombi in veins (eg, deep venous thrombosis [DVT]), arteries (eg, myocardial infarction, ischemic stroke), or cardiac chambers. Thrombi can obstruct blood flow at the site of formation or detach and embolize to block a distant blood vessel (eg, pulmonary embolism, embolic stroke). Acquired conditions are usually a result of surgery, trauma, medications or a medical condition that increases the risk of thrombotic disorders

According to the invention, thrombotic disorders include thrombi present in hereditary hemorrhagic telangiectasia (HHT), prothrombin gene mutation, deficiencies of natural proteins that prevent clotting such as antithrombin, protein C and protein S; elevated level of Factor of coagulation such as factor VII, factor IX and XI; factor V Leiden defect; abnormal fibrinolytic system such as hypoplasminogenemia, dysplasminogenemia and elevation in levels of plasminogen activator inhibitor (PAI-1); dysfibrinolysis; central venous catheter placement; restenosis from stents; obesity; hypercoagulability in pregnancy; antiphospholipid antibody syndrome; cancer; homocystinemia; sticky Platelet Syndrome; pulmonary embolism (PE), Myeloproliferative disorders such as polycythemia vera or essential thrombocytosis; Paroxysmal nocturnal hemoglobinuria (PNH); iatrogenic thromboembolic disorders such as heparin-induced thrombocytopenia (HIT) or thromboembolism induced by haemophilia treatment (emicuzimab, fistusiran,..) ; Inflammatory bowel syndrome such as ulcerative colitis and Chrohn's disease; acquired immune deficiency syndrome (AIDS); coronavirus disease such as SARS-CoV-2 infection (COVID-19 infection); nephrotic syndrome; thrombosis such as artery thrombosis, acute microthrombosis, distal microvascular thrombosis, deep vein thrombosis (DVT), Paget-Schroetter disease, Budd-Chiari syndrome, portal vein thrombosis, renal vein thrombosis, cerebral venous sinus thrombosis, jugular vein thrombosis, and cavernous sinus thrombosis; limn ischemia; sepsis; anemia; sickle-cell disease; cerebral malaria; embolism such as pulmonary embolism and cerebral embolism; and cardiovascular disease such as cardiovascular disease such as cerebrovascular ischemia, acute cerebral infarction, stroke, ischemic stroke, hemorrhagic stroke, aneurysm, mild cognitive impairment (MCI), transient ischemic attacks (TIA), myocardial infarction (or heart attack), atrial fibrillation, corony artery disease, congestive heart failure and the placement of prosthetic heart valves.

In some embodiments, the thrombotic disorders are selected from the group consisting of but not limited to in thrombi present in hereditary hemorrhagic telangiectasia (HHT), embolism (pulmonary and cerebral), COVID-19 infection and cardiovascular disease.

As used herein, the term "hereditary hemorrhagic telangiectasia" (HHT), also known as Olser-Weber-Rendu syndrome, has its general meaning in the art and refers to an inherited disorders causing abnormal blood vessel formation, known as arteriovenous malformations (AVMs), between arteries and vein. More than 80% of all cases of HHT are due to mutations in either ENG or ACVRL1, which cause HHT1 and HHT2, respectively. Hereditary Hemorrhagic Telangiectasia (HHT) is characterized by overwhelming bleeding such as nosebleeds, acute and chronic digestive tract bleeding. However, as explained in Gaetani et al, Journal of Clinical Medicine, 2020, patients afflicted with HTT require also antithrombotic therapy (AT). For instance, they may have coronary artery disease (CAD), venous thromboembolism (VTE), or atrial fibrillation (AF). AT was generally well tolerated, with no fatal bleedings and no significant changes in hemoglobin levels.

As used herein, the term "embolism" refers to lodging of an embolus, a blockage-causing piece of material, inside a blood vessel. The embolus may be a blood clot (thrombus), a fat globule (fat embolism), a bubble of air or other gas (gas embolism), or foreign material. There are different types of embolism, in the context of the invention, the embolism is caused by a blood clot and is selected from the group consisting of but not limited to: arterial embolism, venous embolism or paradoxical embolism. Typically, the arterial embolism can cause occlusion in any part of the body. It is a major cause of infarction (tissue death from blockage of the blood supply). An embolus lodging in the brain from either the heart or a carotid artery most likely be the cause of a stroke due to ischemia. Typically, the venous embolism refers to an embolus formed in a systemic vein that will always impact the lungs, after passing through the right side of the heart. This will form a pulmonary embolism that will result in a blockage of the main artery of the lung and can be a complication of deep-vein thrombosis. The most common sites of origin of pulmonary emboli are the femoral veins. The deep veins of the calf are the most common sites of actual thrombi. Typically, the venous embolism is pulmonary embolism or cerebral embolism.

As used herein, the term "Cardiovascular disease" also known as or "arteriovascular disease" has a general term used to classify numerous conditions affecting the heart, heart valves, blood, and vasculature of the body and encompasses any disease affecting the heart or blood vessels, including, but not limited to, Metabolic Syndrome, Syndrome X, atherosclerosis, atherothrombosis, coronary artery disease, stable and unstable angina pectoris, stroke, diseases of the aorta and its branches (such as aortic stenosis, thrombosis or aortic aneurysm), peripheral artery disease, peripheral vascular disease, cerebrovascular disease, and including, without limitation, any transiently or permanently ischemic arteriovascular event. Arteriovascular disease as used herein is meant to most commonly refer to the ischemic or pro-ischemic disease, rather than generally to non-ischemic disease. As used herein, "atherosclerosis" and "atherothrombosis" refer to systemic inflammatory disease states associated with complex inflammatory responses to multifaceted vascular pathologies involving inflammatory activation of the endothelium, inflammatory leukocytes as a source of thrombogenic stimuli, smooth muscle cells as a source of procoagulants and amplifier of the inflammatory response during thrombosis, and platelets as mediators of inflammation and thrombosis. Arteries harden and narrow due to build up of a material called "plaque" on their inner walls. As the plaque develops and increases in size, the insides of the arteries get narrower ("stenosis") and less blood can flow through them. Stenosis or plaque rupture may cause partial or complete occlusion of the affected vasculature. Tissues supplied by the vasculature are thus deprived of their source of oxygenation (ischemia) and cell death (necrosis) can occur. "CAD" or "coronary artery disease" is an arteriovascular disease which occurs when the arteries that supply blood to the heart muscle (the coronary arteries) become atherosclerotic, calcified and/or narrowed. Eventually, blood flow to the heart muscle is reduced, and, because blood carries much-needed oxygen, the heart muscle is not able to receive the amount of oxygen it needs, and often undergoes necrosis. CAD encompasses disease states such as acute coronary syndromes (ACS), myocardial infarction (heart attack), angina (stable and unstable), and atherosclerosis and atherothrombosis that occurs in the blood vessels that supply the heart with oxygen-rich blood. "CVD" or "cerebrovascular disease" is an arteriovascular disease in the blood vessels that feed oxygen-rich blood to the face and brain, such as atherosclerosis and atherothrombosis. This term is often used to describe "hardening" of the carotid arteries, which supply the brain with blood. It is a common comorbid disease with CAD and/or PAD (peripheral artery disease). It is also referred to as an ischemic disease, or a disease that causes a lack of blood flow. CVD encompasses disease states such as cerebrovascular ischemia, acute cerebral infarction, stroke, ischemic stroke, hemorrhagic stroke, aneurysm, mild cognitive impairment (MCI) and transient ischemic attacks (TIA). Ischemic CVD is believed to closely relate to CAD and PAD; non-ischemic CVD may have multiple pathophysiologies.

In some embodiments, the thrombotic disorders is stroke.

As used herein the term "stroke" refers to any condition arising from a disruption, decrease, or stoppage of blood or oxygen flow to any part of the brain. In particular, the term "stroke" encompasses, without limitation, ischemic stroke, transient ischemic attack (TIA) and haemorrhagic stroke.

As used herein, the terms "treatment" or "treating" refer to both prophylactic or preventive treatment as well as curative or disease modifying treatment, including treatment of subjects at risk of contracting the disease or suspected to have contracted the disease as well as subjects who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a subject during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a subject during treatment of an illness, e.g., to keep the subject in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

As used herein, a "therapeutically effective amount" is intended for a minimal amount of active agent which is necessary to impart therapeutic benefit to a patient. For example, a "therapeutically effective amount of the active agent" to a patient is an amount of the active agent that induces, ameliorates or causes an improvement in the pathological symptoms, disease progression, or physical conditions associated with the disease affecting the patient. It will be understood that the total daily usage of the compounds and compositions of the present invention will be decided by the attending physician within the scope of sound medical judgment. The specific therapeutically effective dose level for any particular patient will depend upon a variety of factors including the age, body weight, general health, sex and diet of the patient; the time of administration, route of administration, and rate of excretion of the specific compound employed; the duration of the treatment; drugs used in combination or coincidental with the specific polypeptide employed; and like factors well known in the medical arts. For example, it is well known within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 100 mg/kg of body weight per day,

As used herein the terms "administering" or "administration" refer to the act of inj ecting or otherwise physically delivering a substance as it exists outside the body (e.g., the nanobody or polypeptide according to the invention) into the subject, such as by mucosal, intradermal, intravenous, subcutaneous, intramuscular delivery and/or any other method of physical delivery described herein or known in the art. When a disease, or a symptom thereof, is being treated, administration of the substance typically occurs after the onset of the disease or symptoms thereof. When a disease or symptoms thereof, are being prevented, administration of the substance typically occurs before the onset of the disease or symptoms thereof.

In some embodiments, the soluble endoglin is administered intravenously.

In a particular embodiment, the soluble endoglin for use according to the invention may be used in combination with classical treatment of thrombotic disorders.

Thus, the invention refers to a method for preventing or treating thrombotic disorders in a subject in need thereof, comprising administering to said subject i) an effective amount of the soluble endoglin and ii) a classical treatment, as a combined preparation for treating thrombotic disorders.

As used herein, the term "classical treatment of thrombotic disorders" refers to any compound, natural or synthetic, used for the treatment of thrombotic disorders and/or thrombectomy.

According to the invention, compound used for the treatment of thrombotic may be selected in the group consisting of vitamin K antagonist such as coumarin, warfarin, acenocoumarol, phenprocoumon, atromentin, fluindione and phenindione; heparin and derivative substance such as enoxaparin, dalteparin, nadroparin and tinzaparin; synthetic pentasaccharide inhibitors of factor Xa such as fondaparinux, idraparinux and idrabiotaparinux; directly acting oral anticoagulants such as dabigatran, rivaroxaban, apixaban, edoxaban, and betrixaban, direct thrombin inhibitors such as hirudin, lepirudin, bivalirudin, argatroban and dabigatran; antithrombin protein; batroxobin; hementin; tissue plasminogen activator (tPA); recombinant tissue plasminogen activator (rtPA) such as alteplase, reteplase, urokinase and tenecteplase; streptokinase; anistreplase; platelet aggregation inhibitor such as clopidogrel, prasugrel, ticagrelor, aspirin, triflusal, cangerlor, ticlopidine, cliostazol, vorapaxar, abciximab, eptifibatide, tirofiban, dipyridamole, thromboxane inhibitors and terutroban; platelet GPVI inhibitors such ACT017 and Revacept ; inhibitor of P-selectin such as Crizanlizumab activator of protein C such as AB002 (WE Thrombin) and soluble thrombomodulin (BDCA-3); or recombinant activated protein C (APC).

As used herein, the term "thromboectomy" has its general meaning in the art and refers to an interventional procedure of removing a blood clot (thrombus) from a blood vessel. It is commonly performed in the cerebral arteries (interventional neuroradiology). The stent-retriever thromboectomy can be performed with general anesthesia or under conscious sedation in an angiographic room. A system of coaxial catheters is pushed inside the arterial circulation, usually through a percutaneous access to the right femoral artery. A microcatheter is finally positioned beyond the occluded segment and a stent-retriever is deployed to catch the thrombus; finally, the stent is pulled out from the artery, usually under continuous aspiration in the larger catheters. A different technique for thrombectomy in the brain is direct aspiration. It is performed by pushing a large soft aspiration catheter into the occluded vessel and applying direct aspiration to retrieve the thrombus; it can be combined with the stent-retriever technique to achieve higher recanalization rates.

As used herein, the terms "combined treatment", "combined therapy" or "therapy combination" refer to a treatment that uses more than one medication. The combined therapy may be dual therapy or bi-therapy.

The medications used in the combined treatment according to the invention are administered to the subject simultaneously, separately or sequentially.

As used herein, the term "administration simultaneously" refers to administration of 2 active ingredients by the same route and at the same time or at substantially the same time. The term "administration separately" refers to an administration of 2 active ingredients at the same time or at substantially the same time by different routes. The term "administration sequentially" refers to an administration of 2 active ingredients at different times, the administration route being identical or different

### Pharmaceutical composition of the invention

According to the invention, the soluble endoglin of the present invention is administered to the subject in the form of a pharmaceutical composition.

Thus, the invention refers to a pharmaceutical composition comprising an soluble endoglin for use for reducing or preventing thrombus formation in a subject in need thereof. The invention also refers to a pharmaceutical composition comprising an soluble endoglin for use for treating thrombotic disorders in a subject in need thereof.

"Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

Solutions comprising inhibitors of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The soluble endoglin can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compounds in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. Some variation in dosage will necessarily occur depending on the condition of the subject being treated. The person responsible for administration will, in any event, determine the appropriate dose for the individual subject.

In addition to the inhibitors of the invention formulated for parenteral administration, such as intravenous or intramuscular injection, other pharmaceutically acceptable forms include, e.g. tablets or other solids for oral administration; liposomal formulations; time release capsules; and any other form currently used.

Pharmaceutical compositions of the invention may include any further agent which is used in the treatment of thrombotic disorders.

In one embodiment, said additional active agents may be contained in the same composition or administrated separately.

In another embodiment, the pharmaceutical composition of the invention relates to combined preparation for simultaneous, separate or sequential use in the prevention and treatment of thrombotic disorders.

The invention will be further illustrated by the following figures and examples. However, these examples and figures should not be interpreted in any way as limiting the scope of the present invention.

### FIGURES:

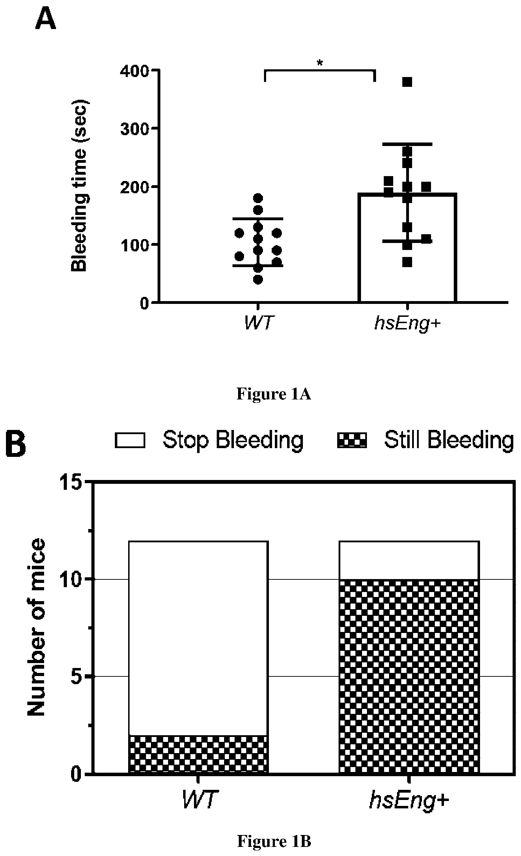

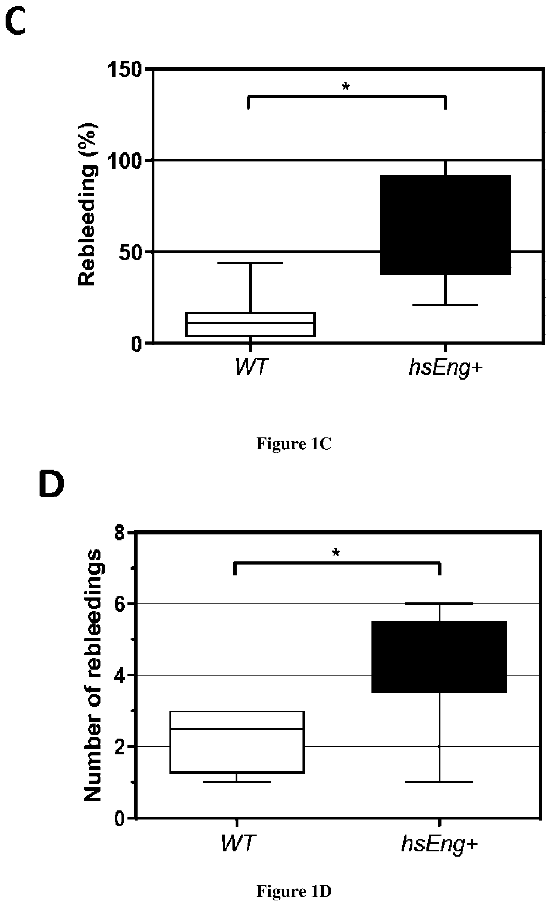

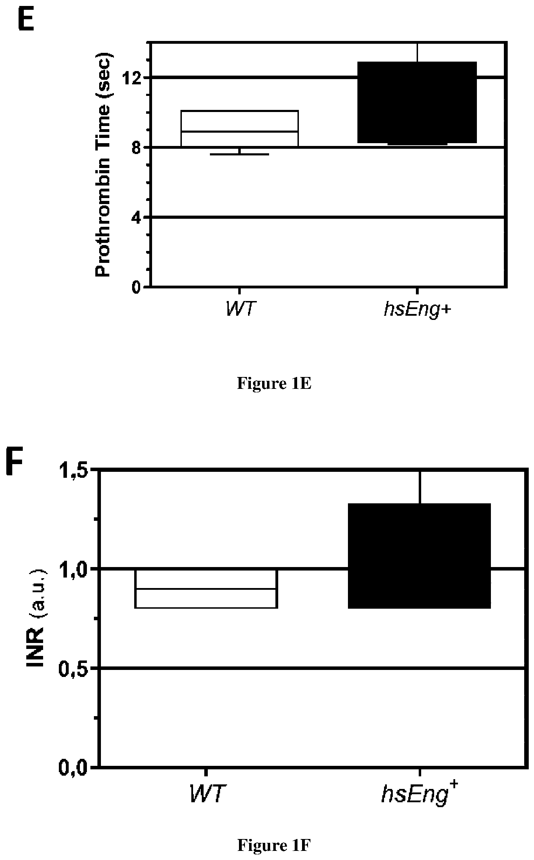

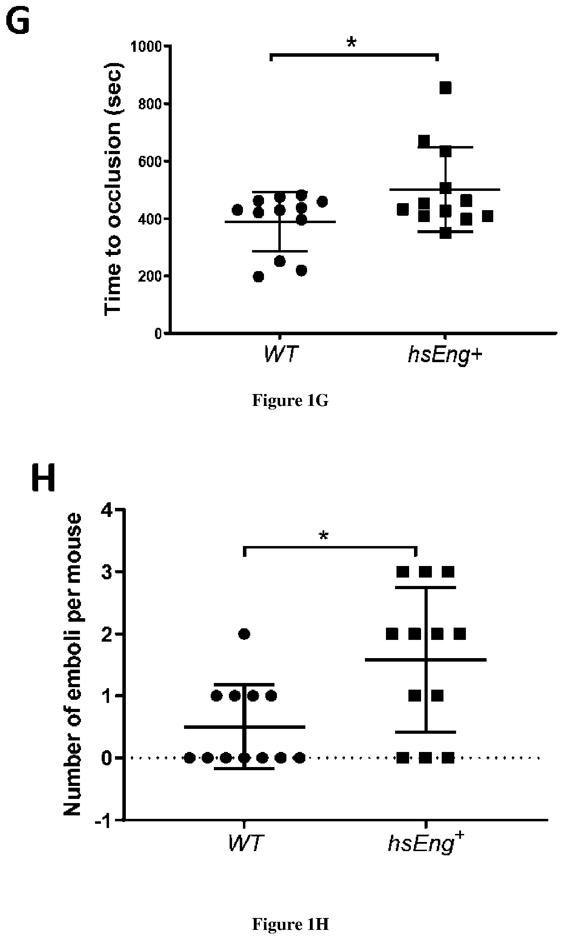

**Figure 1****: Hemostasis assays in *WT* and *hsEng+* mice. A.** Tail bleeding time, corresponding to the first cessation of bleeding, in *WT* (n=12) and *hsEng+* mice (n=12) (*p<0.05). **B.** Final Bleeding. At the end of the experiment (after 30 min) 10 out of 12 *WT* animals stopped to bleed, while 10 out of 12 *hsEng+* mice were still bleeding (*p<0.05). **C.** In *hsEng+* mice, 60% of animals showed rebleeding *versus* 20% of *WT* mice (*p<0.05) and **(D)** the number of rebleedings in *hsEng+* mice was twice that of controls (*p<0.05). No significant differences were found between *WT* and *hsEng+* mice considering prothrombin time **(E)** or the International Normalized Ratio (INR) **(F);** a.u., arbitrary units. **G,H.** Thrombus formation after FeCl3 administration on carotid artery. **G.** Time to final occlusion (blood flow arrest) in *WT* (n=12) and *hsEng+* (n=12) mice (*p<0.05). **H.** A higher number of emboli per mice were found in *hsEng+* mice compared to control animals (*p<0.05).

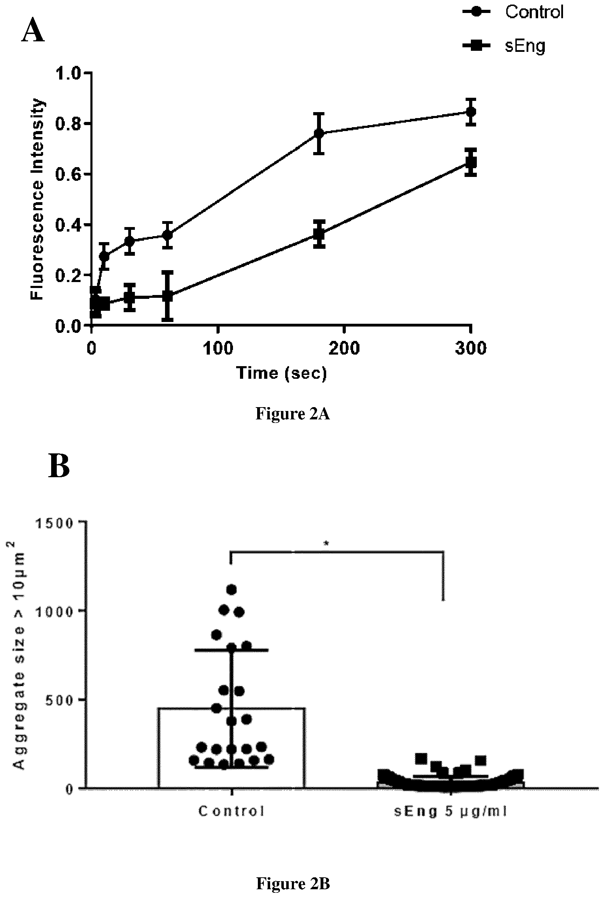

**Figure 2****. Effect of sEng on human platelet adhesion under flow.** Total blood was stained with calcein, loaded on collagen-coated chambers, preincubated with vehicle (control) or with sEng (5 µg/mL), and then perfusion was carried out for 5 min (300 sec). **A.** Fluorescence quantification of different blood donors (n=5) showed that aggregation of platelets occurred more slowly in the presence of sEng (5 µg/mL) than in controls. **B.** Quantification of aggregate size (n=5 different blood donors) showed that sEng-treatment (5 µg/mL) resulted in the formation of more numerous but smaller aggregates compared to control conditions. Indeed, control aggregates frequently reached a size >500 µm2, while in the sEng condition platelets remained disperse and barely formed aggregates larger than the cut-off (10µm²).

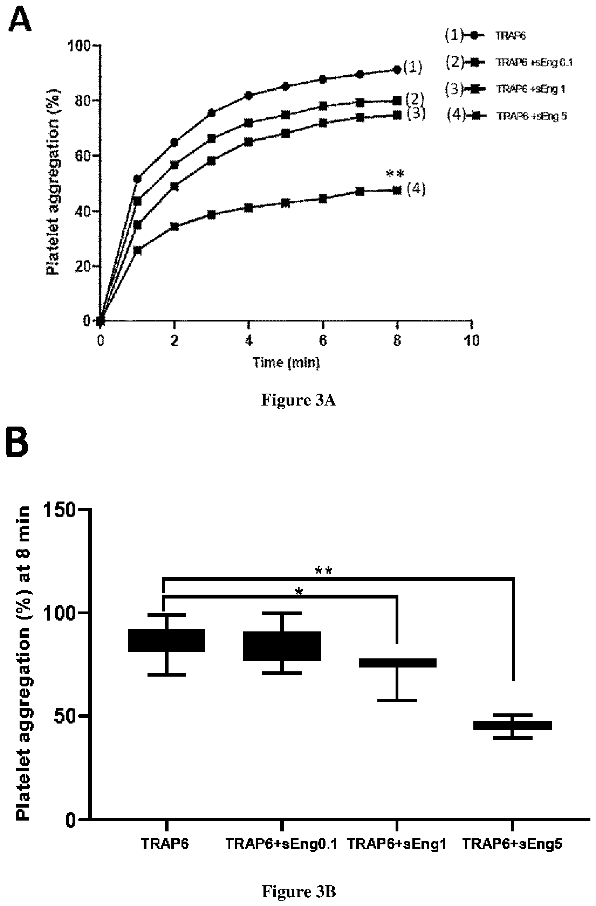

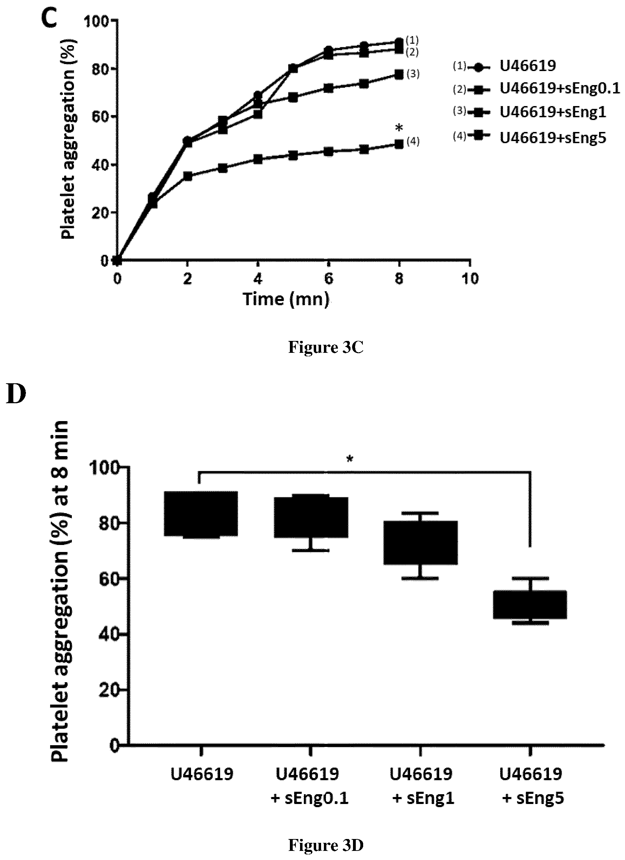

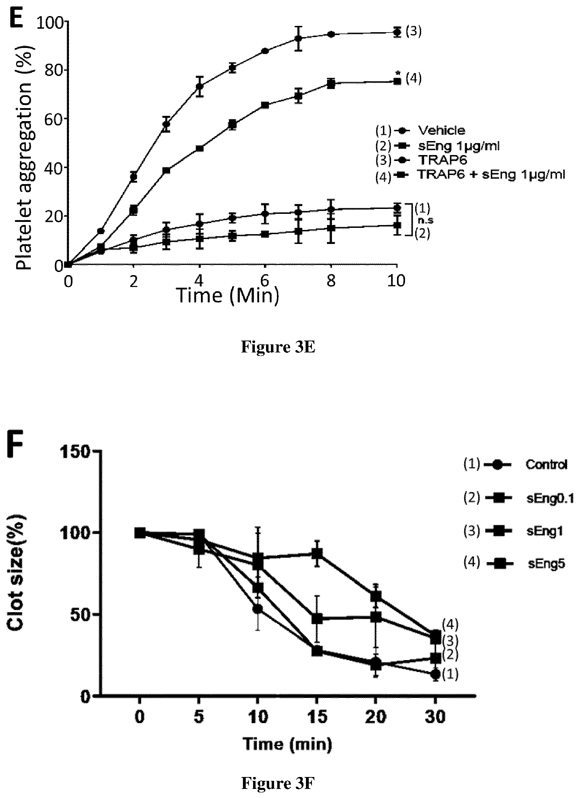

**Figure 3****. Platelet aggregation in microplates and thrombus retraction are inhibited by sEng. A-B.** Human washed platelet (3x10⁸ platelets/mL) were preincubated with vehicle or sEng (0.1, 1 or 5 µg/mL) before addition of 1 µM TRAP6. Aggregations were measured under stirring in a microplate reader set at 37°C. **A.** Aggregation kinetics with different concentrations of sEng. **B.** Quantification of n=10 different donors at 8 min time-point. A significant reduction of aggregation was observed at 1 µg/mL sEng (*p<0.05) and 5 µg/mL sEng (*p<0.01). **C,D.** Platelet aggregation in microplates using the agonist U46619 (0.5 µM). The experimental design was the same as described for TRAP6 (panels **A-B). C.** Kinetics analysis with different concentrations of sEng. **D.** Aggregation was significantly reduced with 5 µg/mL sEng (n=5; **p<0.01). **E.** Aggregation kinetics with buffer, sEng, TRAP6 and sEng+TRAP6. The graph shows that sEng is not able to induce platelet aggregation. **F.** Clot retraction was studied from 0 to 30 minutes after adding thrombin and Ca₂₊ to washed platelets maintained at 37°C with fibrinogen (0.5 mg/mL) in the presence or not (control) of various concentrations of sEng. Kinetic analysis of the retraction experiment expressed as a percentage of the clot size at time 0 (n=5 different donors).

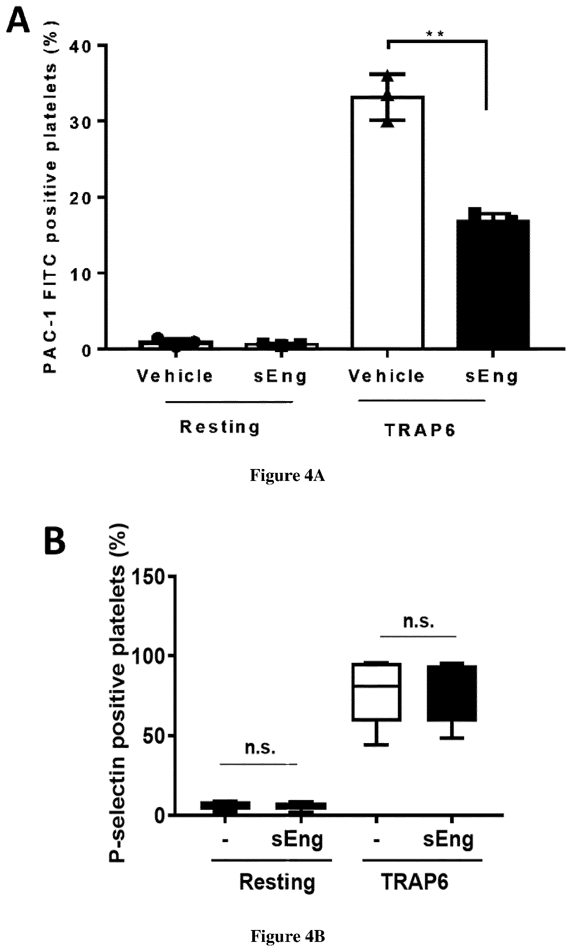

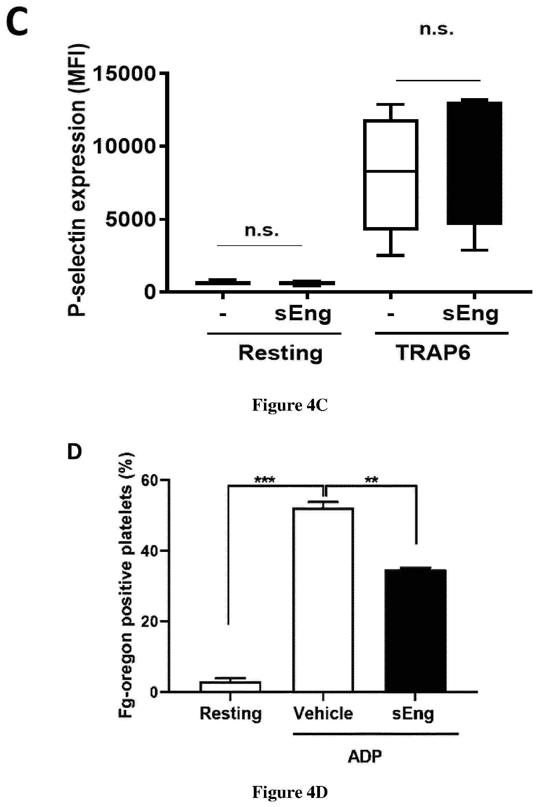

**Figure 4****. sEng does not interfere with platelets activation, but interacts with αIIbβ3 preventing PAC-1 and fibrinogen binding.** Human washed platelets, resting or activated by 2µM TRAP6 or 50 µM ADP, were treated with or without sEng (25µg/mL = 208 nM), as indicated. Then, samples were incubated with (A) FITC-conjugated PAC-1 **(A),** a mix of FITC-conjugated anti-CD62 (anti-P-selectin) and PE-conjugated anti-CD41 antibodies (to determine total platelets) **(B),** or with Oregon Green^{®} 488 fibrinogen **(D)** and analyzed by flow cytometry using a BD Accuri C6 flow cytometer (BD Biosciences). Expression of P-selectin was analyzed by Accuri flow cytometer and expressed as percentage of platelet expressing P-selectin **(B)** and as intensity of P-selectin expression (mean fluorescence intensity; MFI) **(C).** The percentage of positive platelets is displayed in each case. (n=6). (**p<0.01; ***p<0.001; n.s., non-significant)

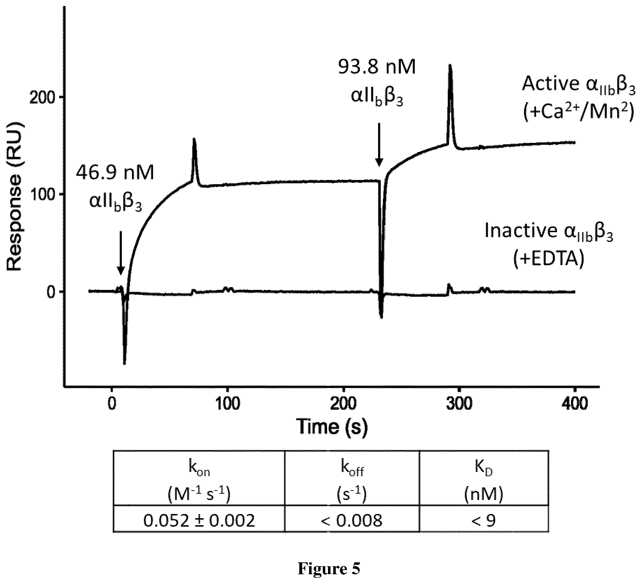

**Figure 5****: Surface plasmon resonance analysis and modeling (Docking) of endoglin and integrin αIIbβ3 interactions.** A. Binding analysis of sEng-Strep-tag^{®}II (sEng-Strep) and integrin αIIbβ3 by surface plasmon resonance. Top, Sensorgram corresponding to the first two consecutive injections of the ligand (integrin αIIbβ3) over the streptavidin sensor chip immobilized with sEng-Strep. The concentration of integrin αIIbβ3 at each injection is indicated. Binding association (kon) and dissociation (koff) phases are shown by arrows. Bottom, apparent kinetic and equilibrium dissociation binding parameters for the interaction calculated by nonlinear regression.

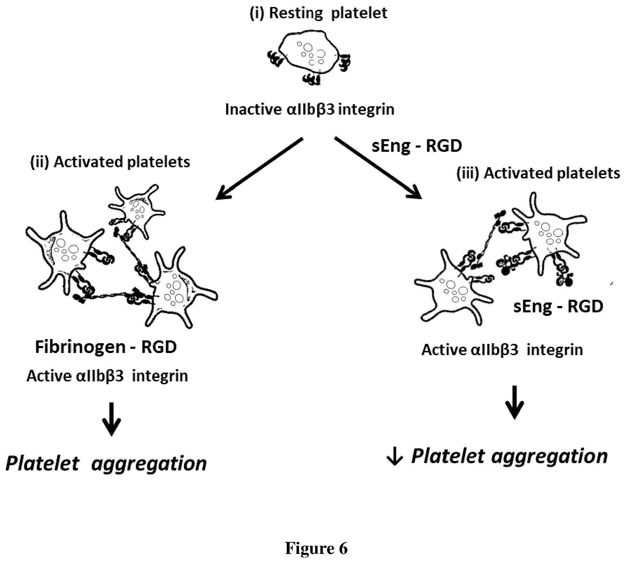

**Figure 6****. Hypothetical mechanism of sEng-induced inhibition of platelet aggregation.**
   Resting platelets expose inactive integrin αIIbβ3 on their surface (i), but after platelet stimulation, integrin αIIbβ3 is activated enabling the formation of fibrinogen-αIIbβ3 interactions mediated by the RGD motif leading to platelet aggregation (ii). sEng binds to αIIbβ3 via its RG motif, inducing a destabilization of the thrombus by "interfering" with fibrinogen-αIIbβ interactions and thus inhibiting platelet aggregation..

### EXAMPLE:

### Material & Methods

### Soluble endoglin

Recombinant human endoglin/CD105 (sEng; R&D Systems, #1097-EN/CF) was used for functional in vitro assays with platelets. For measuring protein-protein interactions by surface plasmon resonance, a streptavidin-tagged construct (sEng-Strep-tag^{®}II) of soluble endoglin was used.

### Transgenic mice

A mouse line overexpressing human sEng (amino acids 26-437) driven by a ubiquitous actin promoter on a CBAxC57BL/6J background (hsEng+) was generated at the Genetically Modified Organisms Generation Unit (University of Salamanca, Spain), as previously described8. Progeny was screened for endoglin transgene by PCR analysis of tail DNA. Studies reported here were performed in the F7 generation. The levels of human sEng in plasma were measured using a specific kit for human soluble endoglin (R&D). Because these transgenic animals show variable levels of sEng in plasma, a minimum threshold of 1,000 ng/mL of plasma sEng was established to include the mouse in the study group⁸. The average plasma levels of human sEng in the tested animals were ~1,600 ng/mL, while control littermate mice showed undetectable amounts of sEng8. All animal procedures were conducted in strict compliance with the European Community Council Directive (2010/63/EU) and Spanish legislation (RD1201/2005 and RD53/2013). The protocols were approved by the University of Salamanca Ethical Committee.

### Bleeding time

To determine the tail bleeding time, hsEng+ and wild type (WT) mice were disposed on carpet with a constant temperature of 37°C and anesthetized with isoflurane. Then, tails were transected at 3mm from the tip and immediately immersed in PBS at 37°C^{5,20}. The time of the first cessation of bleeding was recorded as well as the percentage of animals with rebleedings. The absence of rebleeding for 5 min was considered as cessation of bleeding, whereas mice that were still bleeding after 30 min were scored without arrest. For these last mice, bleeding arrest was obtained by clamping the tail for 5 min.

### Hematologic analysis

Blood samples were obtained from the jugular vein of anesthetized mice in tubes containing 1 mM EDTA. Blood samples were centrifuged at 1,600g for 15 min at 4°C, and plasma was collected. Concentration of human sEng was determined by Quantikine Human Eng/CD105 (R&D Systems, Minneapolis, MN, USA). Prothrombin time and international normalized ratio (INR) were quantified using a portable coagulometer (INRatio^{®}2, Alere). All the assays were performed on 24 mice (n =12 *WT* and n=12 *hsEng*^{*+*)}*.*

### Ferric chloride (FeCl3)-induced carotid artery murine thrombosis model

FeCl3-induced vascular injury is a widely used model of occlusive thrombosis that involves platelet activation and aggregation. This model is based on redox-induced endothelial cell injury²¹. Briefly, a 3 cm incision was performed in the mouse neck and the carotid artery was isolated from the surrounding tissues. Then, a filter paper (1x2mm) soaked in FeCl3 solution (7.5%) was topically deposited for 1 min and 30 sec on the carotid artery, and flux and thrombus formation were monitored in real-time using a Transonic^{®} probe (Transonic Systems Inc., Ithaca, 138 NY). After an initial decrease of the flow, emboli number per mouse was detected considering for each mouse a recovery of the flow >20% (data not shown).

### Human blood donors

Human blood from healthy donors were obtained from the French organization "Etablissement Français du sang (EFS)" (convention C CPSL UNT N° 12/EFS/064) and collected either in BD Vacutainer^{®} tubes containing ACD solution (5.7 mM citric acid, 11.2 mM trisodium citrate, and 20 mM dextrose, final concentrations) or tubes containing a solution of 3.2% sodium citrate.

### In vitro thrombus formation under flow conditions

Aggregation under flow condition was performed as described²². Briefly, total citrated blood was stained with 1 µg/mL calcein (Invitrogen) and this labeling was shown neither to induce nor to alter platelet activation. A total of ten different blood donors were used. Thrombus formation was evaluated in a whole blood perfusion assay on Cellix Vena8 chambers (V8CF-400-100-02P10, tebu-bio), previously coated with a fibrillar collagen matrix (50 µg/mL, Stago) at a shear stress of 56 dynes/cm2 to simulate arteriole flux²³ and then recorded for 5 min. Throughout the perfusion, the same field was observed to monitor platelet adhesion and aggregation. At the end of the infusion, 5 fields were analyzed and the averaged values for each donor (n=5: control vs 5µg/mL sEng) were calculated. Quantification was done by measuring the calcein fluorescence intensity. Thrombus formation was quantified (size cutoff =10 µm2) using Image J software and the corresponding graph elaboration was performed by Graph-Pad Prism software.

### Washed platelet preparation

Whole blood collected on ACD tubes was centrifuged for 11 min at 216g, 22°C to obtain platelet-rich plasma (PRP). PRP was diluted with washing buffer (103 mM NaCl, 5 mM KCl, 2 mM CaCl₂,1 mM MgCl₂, 5 mM glucose and 36 mM citric acid; pH 6.5) containing the platelet inhibitor PGE1 (0.2 µM) and the ADP scavenger apyrase (1 U/mL; Sigma-Aldrich). The PRP suspension was centrifuged for 12 min at 1,240g, 22°C to pellet the platelets. This washing step was repeated once with a washing buffer containing PGE1 and apyrase²⁴. Platelets were finally resuspended at 3.10⁸ platelets/mL in reacting buffer (10 mM HEPES, 140 mM NaCl, 3 mM KCl, 5 mM NaHCO₃, 5 mM MgCl2, 10 mM glucose, pH 7.35) in the presence of 2 mM CaCl₂. This reacting buffer was used as a negative control (vehicle) in sEng experiments.

### Flow cytometry

Human washed platelets (30,000/µL), resting and activated by 50 µM ADP (Kordia), were incubated for 30 min at 37°C with 570 nM Oregon Green^{®} 488 fibrinogen (Invitrogen), and with or without 25 µg/mL sEng (208 nM) in 25 µL of Tyrode's buffer, after which platelet bound Oregon green-fibrinogen was measured by flow cytometry using a BD Accuri C6 flow cytometer (BD Biosciences).

The expression of P-selectin on platelets was assessed using washed platelets (3.108 platelets/mL) pre-incubated for 2 min with vehicle or sEng (5 µM), followed by incubation (without shaking) with PBS (resting) or thrombin receptor activating peptide-6 (TRAP6, Bachem)(2 µM). After 5 min, diluted samples (1: 10 in reacting buffer complemented with 0.3% BSA) were incubated with a mix of FITC-anti-CD62P (BD Pharmingen) and PE-anti-CD41 (Beckman Coulter) antibodies, or corresponding irrelevant antibodies. After 10 min in the dark and a further 1/10 dilution, the samples were immediately analyzed. Platelets were gated on the basis of CD41 expression. P-selectin expression was analyzed using an Accuri (Becton Dickinson) flow cytometer. The results are expressed as percentage of platelet expressing P-selectin or PAC-1 at the surface .

Platelets aggregation induced by ADP agonist allows to measure fibrinogen affinity for platelet integrin α2b-β3. This interaction was found reduced adding sEng.

### Platelet aggregation in microplates and aggregometry

Human washed platelets (3.10⁸/mL) were preincubated in the presence of vehicle or sEng at 0.1, 1, or 5 µg/mL (R&D Systems, #1097-EN/CF) for 2 min at 37°C under stirring in wells of a 96-169 well microplate (half-area flat bottom; Greiner Bio-one, Frickenhausen, Germany). Then, aggregation was induced by adding 1µM of thrombin receptor activating peptide-6 (TRAP6, Bachem) or 0.5µM of the thromboxane A2 receptor agonist U46619 (Calbiochem, Merck, Darmstadt, Germany)²⁵. Extent of platelet aggregation was expressed as the percentage of maximal aggregation calculated as the change in absorbance at 405 nm relative to the absorbance in resting condition and after full platelet aggregation obtained with TRAP6 10µM, as previously described²⁶. Platelet aggregation, performed under the same conditions as above, was also measured using light transmission aggregometry (LTA, ChronoLog Aggregometer Model 700, Chrono-log Corporation, Havertown, PA, USA)²⁷ to confirm microplate results.

### Thrombus retraction

Washed platelets were added in a tube of aggregometry (Bio/Data Corporation) at a final concentration of 2x10⁸ platelets/mL, in the presence of fibrinogen (0.5 mg/mL), as previously described²⁸. The retraction was performed in the presence of various concentrations of sEng (0.1, 1 or 5µg/mL) or vehicle upon preincubation at 37°C for 5 min. Then, a solution of thrombin (Biopharm) and Ca²⁺ (2 IU/mL and 2mM final concentrations, respectively) was added. The tubes were incubated at 37°C during 30 min, and photographed every 5 minutes for clot sizing. The two-dimensional size of retracted clots was quantified with the NIH ImageJ software (http://rsb.info.nih.gov/ij/) by tracing their 2D contours on the photographs. Retraction was expressed as a percentage [(final clot size/initial clot size)x100].

### Surface plasmon resonance binding analysis

The association between sEng-Strep-tag^{®}II and integrin αIIbβ3 was analyzed by surface plasmon resonance (SPR) on a streptavidin (SA) biosensor (Cytiva) at 25°C. First, we immobilized resonance units (RU) of sEng-Strep-tag^{®}II onto the test flow cell of a SA chip through the C-terminal StrepII-tag to provide a sEng concentration on the surface of ~10 µM. The reference flow cell was not modified. Association was then tested in a single-cycle kinetics experiment by injecting increasing concentrations of purified integrin αIIbβ3 (R&D Systems #7148-A2-025 at 46.9, 93.8, 188, 375, and 750 nM) at 30 µL/min (contact time for the analyte or association time was 60 s) in 10 mM HEPES-NaOH, pH 7.4, 150 mM NaCl, and 0.01% (w/v). Polysorbate 20 (PS20), either supplemented with 1 mM MnCl2 and 1 mM CaCl2 or after extensive dialysis against EDTA to remove residual divalent cations in the protein preparation. Integrin αIIbβ3 (25 µg per experiment) was resuspended to 0.2 mg/mL (~1 µM) in the appropriate running buffer before use. Analysis was performed with the BiaEvaluation version 3.0.2 software (Cytiva) and the Anabel webserver.

### Computational analysis

To access the atomistic details of the interaction between sEng and αIIbβ3, we used molecular modeling to generate complexes, as reported²⁹⁻³⁷. For the modelling of αIIbβ3, the well described active and inactive conformations of integrins were used (Takagi and Springer, 2002; Estevez et al., 2015) while for the endoglin modelling, the reported dimeric structure was applied (Saito et al., 2017).

### Statistics

Data were subjected to statistical analysis, and results are shown as mean±SD. Differences in mean values between 2 groups were analyzed with the Student's t-test. Comparisons of several groups were done by 1-way (a single variable) or 2-way (two variables) ANOVA. Then, a posteriori t test was used for group comparisons (GraphPad Software).

### Results

### sEng regulates bleeding and thrombus formation in mice

In order to assess the role of sEng in hemostasis, we first analyzed bleeding parameters and thrombus formation in a previously reported transgenic mouse line which overexpresses human sEng (*hsEng⁺*)⁸*.* No differences existed between *hsEng+* and *WT* mice in terms of hematology parameters such as platelet count (data not shown). We observed that *hsEng+* mice had significantly longer bleeding times, either considering the first cessation of bleeding (Figure 1A) or total bleeding (bleeding + rebleeding), since at 30 min 10 out of 12 *hsEng⁺*mice continued to bleed compared to 2 out of 10 for the *WT*(*p<0.05) (Figure 1B). Furthermore, we found that the number of animals undergoing rebleedings was significantly higher in hsEng+ mice than controls (p<0.05) (Figure 1C). In addition, the total number of rebleedings per mouse was markedly increased in *hsEng+* mice compared to control animals (p<0.05) (Figure 1D). Importantly, no significant differences existed in prothrombin time or International Normalized Ratio (INR) between hsEng+ and WT mice (Figure 1E-F). The impact of increased concentrations of sEng on hemostasis was also evaluated in vivo using the model of carotid artery thrombosis. Thus, hsEng+ mice showed a significant longer time to occlusion than WT (500±147 vs 388±103 sec, respectively; p<0.05) (Figure 1G). Moreover, emboli were more frequent in *hsEng+* mice compared to WT, either considering the number of emboli per mouse (mean 1.6 ± 0.8 vs 0.5 ±0.5 respectively; p=0.01 (Figure 1H) or the number of mice that had at least one embolic event. Indeed, the analysis of full flow recovery after partial closure showed that 38% of WT animals presented at least one reopening event after partial closure and this percentage increased to 78% in *hsEng+* mice, suggesting that thrombi are less stable in hsEng+ than in WT mice. Taken together, these results support a potential effect of sEng on primary hemostasis in vivo.

### sEng reduces thrombus formation under flow conditions in vitro

The effect of sEng on thrombus formation was tested in vitro under flow conditions, using human whole blood supplemented or not with sEng (5 µg/mL) and perfused on collagen-coated chambers. As shown in Figure 2A, sEng did not prevent initial adhesion of platelets to collagen but decreased the size of the aggregates. Indeed, in the presence of sEng, aggregates were smaller than in controls (mean 30µm vs 200 µm p<0.001) (Figure 2A-B). Similar results were obtained using 1 µg/mL sEng, although the effect at this concentration was lower (*p<0.05) (data not shown).

### sEng inhibits platelet aggregation and thrombus retraction

Human washed platelets, preincubated with vehicle or with various concentrations of sEng, were activated by TRAP6 (1 µM) and aggregation was monitored in microplates as reported^{25,38}. sEng induced a dose dependent inhibition of aggregation that was statistically significant at 1 µg/mL of sEng and reached 50% inhibition at 5 µg/mL sEng (Figure 3A,B). Compared to vehicle-treated platelets, TRAP6 at 1 µM induced a strong aggregation which reached, at least, 80% with all donors tested. sEng alone did not induce platelet aggregation at the concentrations used (Figure 3E). On the contrary, sEng induced a dose dependent inhibition of TRAP6-induced aggregation that was statistically significant at 1 µg/m 240 L (*p<0.05) of sEng and reached 50% inhibition at 5 µg/mL sEng (**p<0.01) (Figure 3A,C,E).

Moreover, to rule out that the sEng-induced inhibition of aggregation was not restricted to TRAP6-induced activation, the experiment was reproduced using the thromboxane-prostanoid (TP)-receptor agonist U46619 (0.5 µM). As in the case of TRAP6 activation, the aggregation was significantly reduced with 5µg/mL sEng (**p<0.01) (Figure 3C,D). The inhibition of aggregation was also observed by the disappearance of large aggregates in suspension at the end of the reaction (data not shown). These results were confirmed using the reference technique of optical aggregometry (data not shown). To evaluate thrombus retraction, thrombin and Ca²⁺ were added to washed platelets supplemented with fibrinogen (data not shown) in the presence of various concentrations of sEng (0.1 µg/mL, 1 µg/mL or 5 µg/mL) (Figure 3F). Photos were taken every five minutes from 0 to 30 min (data not shown). At 15 min, a significantly impaired clot retraction was observed in the presence of 5 µg/mL sEng compared to control (0), with mean retraction values of 10% vs 70%, respectively (*p<0.01) 245 (Figure 3F). Clot retraction impairement was also significant with 1 µg/mL sEng at 30 min (*p<0.05) (Figure 3F). To determine whether the inhibitory activity of sEng was specific to platelet aggregation and had no direct effect on platelet activation, we next examined the impact of sEng on platelet secretion.

### sEng does not interfere with platelet activation, but interacts with αIIbβ3 preventing fibrinogen binding

We found that sEng decreased the binding of PAC-1 (a monoclonal antibody that specifically recognized activated integrin αIIbβ3), to platelets activated by TRAP6 (Figure 4A; p<0.01), likely by acting on the αIIbβ3 complex. Interestingly, in the same condition, the percentage of positive platelets for P-selectin (a marker of platelet secretion) was not modified in the presence of sEng (Figure 4B). This lack of modulatory activity of sEng on secretion suggests that sEng does not affect platelet activation but possibly PAC-1 binding to integrin. To further test the hypothesis that sEng inhibits aggregation by direct interaction with αIIbβ3, a competition assay between sEng and Oregon Green 488-labelled-fibrinogen on resting or activated platelets was performed. As expected, ADP, a weak platelet agonist, induced activation of αIIbβ3 and thus fibrinogen binding, whereas sEng significantly decreased this binding (Figure 3C; p<0.01).

### Computational analysis provides support for the possible interaction between Eng and integrin αIIbβ3

Taken together, the above results suggest that the modulatory effects of sEng on hemostasis could be mediated by its interaction with the integrin αIIbβ3, which is expressed at high levels in platelets. To investigate this interaction, we analyzed the association between sEng-Strep-tag^{®}II (sEng-Strep) and integrin αIIbβ3 by surface plasmon resonance (SPR) on a streptavidin (SA) biosensor (Cytiva) at 25 °C using single-cycle kinetics. When non-activated integrin αIIbβ3 was flowed over surface-immobilized sEng-Strep, no binding was detected (Figure 5). In contrast, when activated Ca2+/Mn2+-loaded integrin αIIbβ3 was flowed instead, the first (46.9 nM) and second (93.8 nM) injections showed fast binding and slow dissociation phases, which allowed the estimation of approximate binding parameters (Figure 5). The second injection already accomplished 100% binding saturation at 168 RU (maximum theoretical RU = 150 RU). Apparent association (kon = 0.052 ± 0.002 M-1 s-1) and dissociation (koff < 0.008 s-1) kinetic constants were estimated from the complete single-cycle kinetics experiment or the first injection only and used to estimate the apparent equilibrium dissociation constant KD < 9 nM. At greater concentrations of integrin αIIbβ3 (>100 nM), binding either plateaued, indicating binding saturation on the SA chip, or was artificially reduced through nonspecific binding to the reference flow cell.

The above data demonstrate the direct interaction between sEng and integrin αIIbβ3 and this association was further analyzed *in silico.* Docking analysis showed that sEng and active (open conformation) or inactive (closed conformation) integrin αIIbβ3 could form low energy stable complexes (data not shown). As detailed in the method section, we discarded all hits that were not physically relevant because the interaction surface or the number of contact residues were too small to yield a plausible stable docking. Moreover, we analyzed the details of the contact regions, focusing those for which there was a true penetration of one chain into the other and establishing a relevant number of contacts. We first focused on the complex formed by active αIIbβ3 as this form is the one that typically interacts with fibrinogen. We found several possible stable complexes and large clusters of structures exhibiting a direct interaction between one of the RGD motifs of sEng and αIIbβ3 (data not shown). In this condition, the position occupied by RGD is at the interface between αIIb and β3, in the same region where fibrinogen binds. Next, we also investigated the possible interaction between sEng with the closed conformation of inactive αIIbβ3. When looking at the sEng+inactive integrin complex, we found structures with just one arm of sEng engaged in interacting with αIIbβ3, while the other arm remained free of contacts (data not shown). Overall, our *in silico* predictions of a more stable complex between sEng and the activated αIIbβ3 compared to the complex formed with the inactivated αIIbβ3, are in agreement with the strong versus lack of interaction, respectively, detected by SPR (Figure 5).

### Discussion

The study demonstrates that sEng interferes with thrombus formation and stabilization. Thrombus formation is mediated by αIIbβ3 that, on resting platelets, is in an inactive conformation with a low affinity for fibrinogen. Following platelet activation αIIbβ3, switching from low affinity (resting) to high affinity (active) state for ligand binding and thus acquires its platelet aggregation receptor activity resulting in its key role in hemostasis. Recently, we reported the interaction between membrane endoglin of endothelial cells with platelets, involving the endoglin RGD motif and the activated αIIbβ3 of human and murine platelets69. Furthermore, in vitro experiments using human Eng mutated on the RGD sequence (i.e. RGA) supported the role of this motif in the Eng-integrin interaction. Since sEng encompasses the extracellular region of membrane endoglin, including the RGD motif, we hypothesized that sEng is able to bind integrin αIIbβ3 in humans as well as in mice, which entails a series of consequences. Our present study supports this hypothesis since the data demonstrate that sEng intervenes in platelet aggregation, acting as a competitor of fibrinogen during clot formation. This is in agreement with the tight KD value (KD<9 nM) of the sEng/αIIbβ3 interaction compared with that of fibrinogen/αIIbβ3 (KD=50 nM), both measured using the same SPR technique. Indeed, using whole blood aggregation assays in flow conditions we found that sEng reduced aggregation stimulated by strong agonists, such as TRAP6 and the TP-receptor agonist U46619, or a weak agonist such as ADP, without affecting platelet activation, as suggested by its absence of effect on platelet secretion.

Clot retraction, allowing the stabilitization of the aggregates, is the final step during platelet activation which involves the binding of fibrinogen to αIIbβ3 and consequently the outside-in signaling pathways that induce cytoskeletal rearrangement necessary for the retraction. Platelets pull on fibrin strands to consolidate the hemostatic plug and to further allow the blood flow to circulate. Therefore, we also evaluated thrombus retraction in the presence of sEng, providing evidence that sEng delays clot retraction. The inhibitory effect of sEng on ligand binding to αIIbβ3 is also supported by our in vitro assays which demonstrated that sEng binds platelets after stimulation and interferes with fibrinogen and PAC-1 binding without affecting platelet activation.

A two-step mechanism for αIIbβ3 binding to fibrinogen has been suggested: the γ sequence of fibrinogen by itself is able to initiate αIIbβ3 clustering and recruitment of intracellular proteins to early focal complexes, mediating cell attachment, while the RGD motif (on the α chain) subsequently acts as a molecular switch on the β3 subunit to trigger cell spreading. Figure 6 illustrates our hypothesis that sEng inhibits platelet aggregation and induces a destabilization of thrombus by preventing or interfering with fibrinogen-αIIbβ3 bridges. Supporting the involvement of sEng in this process, SPR and computational analyses showed that the interaction sEng-αIIbβ3 occurs when the integrin is in its active form, but not in its inactive form.

The inhibitory effect of sEng was also confirmed *in vivo.* Indeed, we showed that mice overexpressing sEng (*hsEng+*) and displaying a range of circulating endoglin concentration between 1-5 µg/mL bled more than *WT,* present more rebleedings and impairment in bleeding arrest even after 30 min, while no anomalies in coagulation factor levels were observed. Furthermore, after inducing thrombus formation, we found that artery occlusion lasted longer and was less stable in *hsEng+* than in control mice, suggesting that sEng circulating in *hsEng+* mice was implicated in clot formation and stabilization.

Integrin αIIbβ3 is a target of potent anti-platelet agents such as eptifibatide, tirofiban and abciximab. However, due to the associated high bleeding risk, their use is currently limited to acute settings such as during percutaneous coronary intervention or acute coronary syndrome in patients with high thrombotic risk. Therefore, there is a current need to find alternative, anti-platelet and safer drugs. In this context, our results propose that Eng is a physiological ligand of αIIbβ3 and open a new research avenue to study sEng as an anti-platelet drug agent. All in all, our data highlight a beneficial and potential interest of sEng not only to prevent platelet binding to fibrinogen, impairing aggregation and the stability of the thrombus, but also to identify and target platelets with sEng for new therapeutic approaches.

In summary, we described for the first time the potential significance of the interaction between sEng and αIIbβ3 integrin in the thrombo-inflammatory context. This patho-physiological context is controlled by αIIbβ3, which is a platelet-specific integrin that plays a key role in platelet functions, namely in platelet aggregation, a central response in hemostasis and thrombosis.

### REFERENCES:

Throughout this application, various references describe the state of the art to which this disclosure pertains.
1. López-Novoa JM, Bernabeu C. The physiological role of endoglin in the cardiovascular system. Am. J. Physiol. - Heart Circ. Physiol. 2010;299(4): H959-74.
2. Shovlin CL. Hereditary haemorrhagic telangiectasia: Pathophysiology, diagnosis and treatment. Blood Rev. 2010;24(6):203-219.
3. Faughnan ME, Mager JJ, Hetts SW, et al. Second International Guidelines for the Diagnosis and Management of Hereditary Hemorrhagic Telangiectasia. Ann. Intern. Med. 2020; 173(12):989-1001.
4. Albiñana V, Cuesta AM, de Rojas-P I, et al. Review of Pharmacological Strategies with Repurposed Drugs for Hereditary Hemorrhagic Telangiectasia Related Bleeding. J. Clin. Med. 2020;9(6):1766.
5. Rossi E, Pericacho M, Bachelot-Loza C, et al. Human endoglin as a potential new partner 392 involved in platelet-endothelium interactions. Cell. Mol. Life Sci. 2018;75(7):1269-1284.
6. Bennett JS, Berger BW, Billings PC. The structure and function of platelet integrins. J. Thromb. Haemost. 2009;7(SUPPL. 1):200-205.
7. Hawinkels LJAC, Kuiper P, Wiercinska E, et al. Matrix metalloproteinase-14 (MT1-MMP)-mediated endoglin shedding inhibits tumor angiogenesis. Cancer Res. 2010;70(10):4141-4150.
8. Valbuena-Diez AC, Blanco FJ, Oujo B, et al. Oxysterol-induced soluble endoglin release and its involvement in hypertension. Circulation. 2012;126(22):2612-2624.
9. Aristorena M, Gallardo-Vara E, Vicen M, et al. MMP-12, secreted by pro-inflammatory macrophages, targets endoglin in human macrophages and endothelial cells. Int. J. Mol. Sci. 2019;20(12): :3107.
10. Rossi E, Sanz-Rodriguez F, Eleno N, et al. Endothelial endoglin is involved in inflammation: Role in leukocyte adhesion and transmigration. Blood. 2013;121(2):403-415.
11. Rossi E, Smadja DM, Boscolo E, et al. Endoglin regulates mural cell adhesion in the circulatory system. Cell. Mol. Life Sci. 2016;73(8):1715-1739.
12. Rossi E, Bernabeu C, Smadja DM. Endoglin as an adhesion molecule in mature and progenitor endothelial cells: A function beyond TGF-β. Front. Med. 2019;6(JAN):1-8.
13. Gregory AL, Xu G, Sotov V, Letarte M. Review: The enigmatic role of endoglin in the placenta. Placenta. 2014;35(SUPPL):93-99.
14. Pérez-Roque L, Núñez-Gómez E, Rodriguez-Barbero A, et al. Pregnancy-induced high plasma levels of soluble endoglin in mice lead to preeclampsia symptoms and placental abnormalities. Int. J. Mol. Sci. 2021;22(1):1-20.
15. Rathouska J, Jezkova K, Nemeckova I, Nachtigal P. Soluble endoglin, hypercholesterolemia and endothelial dysfunction. Atherosclerosis. 2015;243(2):383-388.
16. Emeksiz HC, Bideci A, Damar Ç, et al. Soluble endoglin level increase occurs prior to development of subclinical structural vascular alterations in diabetic adolescents. JCRPE J. Clin. Res. Pediatr. Endocrinol. 2016;8(3):313-320.
17. Gallardo-Vara E, Gamella-Pozuelo L, Perez-Roque L, et al. Potential Role of Circulating Endoglin in Hypertension via the Upregulated Expression of BMP4. Cells. 2020;9(4):988.
18. Saita E, Miura K, Suzuki-Sugihara N, et al. Plasma soluble Endoglin levels are inversely associated with the severity of coronary atherosclerosis - Brief Report. Arterioscler. Thromb. Vasc. Biol. 2017;37(1):49-52.
19. Cruz-Gonzalez I, Pabón P, Rodríguez-Barbero A, et al. Identification of serum endoglin as a novel prognostic marker after acute myocardial infarction: Molecular Diagnosis. J. Cell. Mol. Med. 2008;12(3):955-961.
20. Mohammed BM, Monroe DM, Gailani D. Mouse models of hemostasis. Platelets. 2020;31(4):417-422.
21. Li W, Nieman M, Sen Gupta A. Ferric chloride-induced murine thrombosis models. J. Vis. Exp. 2016;2016(115):1-12.
22. Hechler B, Dupuis A, Mangin PH, Gachet C. Platelet preparation for function testing in the laboratory and clinic: Historical and practical aspects. Res. Pract. Thromb. Haemost. 2019;3(4):615-625.
23. Paszkowiak JJ, Dardik A. Arterial wall shear stress: Observations from the bench to the bedside. Vasc. Endovascular Surg. 2003;37(1):47-57.
24. Dumas M, Nadal-Wollbold F, Gaussem P, et al. Antiplatelet and antithrombotic effect of F 438 16618, a new thrombin proteinase-activated receptor-1 (PAR1) antagonist. Br. J. Pharmacol. 2012;165(6):1827-1835.
25. Broos K, Feys HB, De Meyer SF, Vanhoorelbeke K, Deckmyn H. Platelets at work in primary hemostasis. Blood Rev. 2011;25(4):155-167.
26. Decouture B, Dreano E, Belleville-Rolland T, et al. Impaired platelet activation and cAMP homeostasis in MRP4-deficient mice. Blood. 2015;126(15):1823-1830.
27. Martin AC, Zlotnik D, Bonete GP, et al. Epinephrine restores platelet functions inhibited by ticagrelor: A mechanistic approach. Eur. J. Pharmacol. 2020;866(August 2019):172798.
28. Egot M, Lasne D, Poirault-Chassac S, et al. Role of oculocerebrorenal syndrome of Lowe (OCRL) protein in megakaryocyte maturation, platelet production and functions: a study in patients with Lowe syndrome. Br. J. Haematol. 2021;192(5):909-921.
29. Saito T, Bokhove M, Croci R, et al. Structural Basis of the Human Endoglin-BMP9 Interaction: Insights into BMP Signaling and HHT1. Cell Rep. 2017;19(9):1917-1928.
30. Llorca O, Trujillo A, Blanco FJ, Bernabeu C. Structural Model of Human Endoglin, a Transmembrane Receptor Responsible for Hereditary Hemorrhagic Telangiectasia. J. Mol. Biol. 2007;365(3):694-705.
31. Frezza E, Martin J, Lavery R. A molecular dynamics study of adenylyl cyclase: The impact of ATP and G-protein binding. PLoS One. 2018;13(4):1-17.
32. Zhu J, Luo BH, Xiao T, et al. Structure of a Complete Integrin Ectodomain in a Physiologic Resting State and Activation and Deactivation by Applied Forces. Mol. Cell. 2008;32(6):849-861.
33. Takagi J, Petre BM, Walz T, Springer TA. Global conformational earrangements in integrin extracellular domains in outside-in and inside-out signaling. Cell. 2002;110(5):599-611.
34. Springer TA, Zhu J, Xiao T. Structural basis for distinctive recognition of fibrinogen γC peptide by the platelet integrin αIIbβ3. J. Cell Biol. 2008;182(4):791-800.
35. Veesler D, Cupelli K, Burger M, et al. Single-particle EM reveals plasticity of interactions between the adenovirus penton base and integrin αVβ3. Proc. Natl. Acad. Sci. U. S. A. 2014;111(24):8815-8819.
36. Pettersen EF, Goddard TD, Huang CC, et al. UCSF Chimera - A visualization system for exploratory research and analysis. J. Comput. Chem. 2004;25(13):1605-1612.
37. Suhre K, Sanejouand YH. ElNémo: A normal mode web server for protein movement analysis and the generation of templates for molecular replacement. Nucleic Acids Res. 2004;32(WEB SERVER ISS.):610-614.
38. Lordkipanidzé M, Lowe GC, Kirkby NS, et al. Characterization of multiple platelet activation pathways in patients with bleeding as a high-throughput screening option: Use of 96-well Optimul assay. Blood. 2014;123(8):11-23.
39. Huang J, Li X, Shi X, et al. Platelet integrin αiIbβ3: Signal transduction, regulation, and its therapeutic targeting. J. Hematol. Oncol. 2019;12(1):1-22.
40. Shattil SJ, Newman PJ. Integrins: Dynamic scaffolds for adhesion and signaling in platelets. Blood. 2004;104(6):1606-1615.
41. Nurden AT, Pillois X, Wilcox DA. Glanzmann thrombasthenia: State of the art and future directions. Semin. Thromb. Hemost. 2013;39(6):642-655.
42. Tronik-Le Roux D, Roullot V, Poujol C, et al. Thrombasthenic mice generated by replacement of the integrin α(IIb) gene: Demonstration that transcriptional activation of this megakaryocytic locus precedes lineage commitment. Blood. 2000;96(4):1399-1408.
43. Swieringa F, Spronk HMH, Heemskerk JWM, van der Meijden PEJ. Integrating platelet and coagulation activation in fibrin clot formation. Res. Pract. Thromb. Haemost. 2018;2(3):450-460.
44. Holmbäck K, Danton MJS, Suh TT, Daugherty CC, Degen JL. Impaired platelet aggregation and sustained bleeding in mice lacking the fibrinogen motif bound by integrin 21 α(IIb)β3. EMBO J. 1996;15(21):5760-5771.
45. Salsmann A, Schaffner-Reckinger E, Kabile F, Plançon S, Kieffer N. A new functional role of the fibrinogen RGD motif as the molecular switch that selectively triggers integrin αIIbβ3-dependent RhoA activation during cell spreading. J. Biol. Chem. 2005;280(39):33610-33619.
46. Burkhart JM, Gambaryan S, Watson SP, et al. What can proteomics tell us about platelets? Circ. Res. 2014;114(7):1204-1219.

## Claims

1. A soluble endoglin for use for reducing or preventing thrombus formation in a subject in need thereof.

2. The soluble endoglin for use according to claim 1, wherein the thrombus is caused by thrombotic disorders.

3. The soluble endoglin for use according to claim 1 or 2, wherein the soluble endoglin comprises or refers to the amino acid sequence of SEQ ID NO: 1 or SEQ ID NO:2.

4. The soluble endoglin for use according to claims 2 or 3, wherein the thrombotic disorder is selected in the group consisting of hereditary hemorrhagic telangiectasia, prothrombin gene mutation, deficiencies of natural proteins that prevent clotting; deficiencies of antithrombin; deficiencies of protein C; deficiencies of protein S; elevated level of Factor of coagulation; abnormal fibrinolytic system; hypoplasminogenemia, dysplasminogenemia; elevation in levels of plasminogen activator inhibitor (PAI-1); dysfibrinolysis; obesity; hypercoagulability in pregnancy; antiphospholipid antibody syndrome; cancer; homocystinemia; sticky Platelet Syndrome; pulmonary embolism (PE), Myeloproliferative disorders polycythemia vera or essential thrombocytosis; Paroxysmal nocturnal hemoglobinuria (PNH); iatrogenic thromboembolic disorders; heparin-induced thrombocytopenia (HIT); thromboembolism induced by haemophilia treatment; inflammatory bowel syndrome; ulcerative colitis; chrohn's disease; acquired immune deficiency syndrome (AIDS); coronavirus disease; nephrotic syndrome; thrombosis; artery thrombosis; acute microthrombosis; distal microvascular thrombosis; deep vein thrombosis (DVT); Paget-Schroetter disease; Budd-Chiari syndrome; portal vein thrombosis; renal vein thrombosis; cerebral venous sinus thrombosis; jugular vein thrombosis; cavernous sinus thrombosis; limn ischemia; sepsis; anemia; sickle-cell disease; cerebral malaria; embolism; pulmonary embolism; cerebral embolism; cardiovascular disease; cerebrovascular ischemia, acute cerebral infarction, stroke, ischemic stroke, hemorrhagic stroke, aneurysm, mild cognitive impairment (MCI), transient ischemic attacks (TIA), myocardial infarction (or heart attack), atrial fibrillation, corony artery disease, and congestive heart failure.

5. The soluble endoglin for use according to claims 4, wherein the thrombotic disorders is selected from the group consisting of but not limited to hereditary hemorrhagic telangiectasia (HHT), pulmonary embolism, cerebral embolism, COVID-19 infection and cardiovascular disease.

6. The soluble endoglin for use according to claims 4, wherein the thrombotic disorders is stroke.

7. The soluble endoglin for use according to claims 1, wherein the thrombus is caused by central venous catheter placement; restenosis from stents or the placement of prosthetic heart valves.

8. The soluble endoglin for use according to any one of claim 1 to 7, wherein the soluble endoglin is used in combination with classical treatment of thrombotic disorders.

9. The soluble endoglin for use according to any one of claim 1 to 8, wherein the soluble endoglin is administered to the subject in the form of a pharmaceutical composition.

## Patentansprüche

1. Lösliches Endoglin zur Verwendung bei Verringerung oder Verhinderung der Thrombusbildung bei einem bedürftigen Subjekt.

2. Lösliches Endoglin zur Verwendung nach Anspruch 1, wobei der Thrombus durch thrombotische Störungen verursacht wird.

3. Lösliches Endoglin zur Verwendung nach Anspruch 1 oder 2, wobei das lösliche Endoglin die Aminosäuresequenz aus SEQ ID-Nr. 1 oder SEQ ID-Nr. 2 umfasst oder sich darauf bezieht.

4. Lösliches Endoglin zur Verwendung nach Ansprüchen 2 oder 3, wobei die thrombotische Störung ausgewählt ist aus der Gruppe bestehend aus hereditärer Teleangiektasie, Prothrombin-Genmutation, Mängeln an natürlichen Proteinen, welche die Gerinnung verhindern; Mängeln an Antithrombin; Mängeln an Protein C; Mängeln an Protein S; erhöhtem Niveau des Gerinnungsfaktors; abnormalem fibrinolytischem System; Hypoplasminogenämie, Dysplasminogenämie; erhöhten Werten des Plasminogen-Aktivator-Inhibitors (PAI-1); Dysfibrinolyse; Adipositas; Hyperkoagulabilität in der Schwangerschaft; Antiphospholipid-Antikörper-Syndrom; Krebs; Homocystinämie; Sticky-Platelet-Syndrom; Lungenembolie (PE), myeloproliferativen Störungen Polycythemia vera oder essentieller Thrombozytose; paroxysmaler nächtliche Hämoglobinurie (PNH); iatrogenen thromboembolischen Störungen; Heparin-induzierte Thrombozytopenie (HIT); durch Hämophilie-Behandlung induzierter Thromboembolie; entzündlichem Darmsyndrom; Colitis ulcerosa; Morbus Crohn; erworbenem Immundefektsyndrom (AIDS); Coronavirus-Erkrankung; nephrotischem Syndrom; Thrombose; Arterienthrombose; akuter Mikrothrombose; distaler mikrovaskulärer Thrombose; tiefer Venenthrombose (DVT); Paget-von-Schroetter-Krankheit; Budd-Chiari-Syndrom; Pfortaderthrombose; Nierenvenenthrombose; cerebral-venöser Sinusthrombose; Jugularvenenthrombose; kavernöser Sinusthrombose; Extremitätenischämie; Sepsis; Anämie; Sichelzellenkrankheit; zerebraler Malaria; Embolie; Lungenembolie; Hirnembolie; kardiovaskulären Erkrankungen; zerebrovaskulärer Ischämie, akutem Hirninfarkt, Schlaganfall, ischämischem Schlaganfall,
hämorrhagischem Schlaganfall, Aneurysma, leichter kognitiver Beeinträchtigung (MCI), transitorischen ischämischen Attacken (TIA), Myokardinfarkt (oder Herzanfall), Vorhofflimmern, Koronararterienerkrankung und kongestiver Herzinsuffizienz.

5. Lösliches Endoglin zur Verwendung nach Ansprüchen 4, wobei die thrombotischen Störungen ausgewählt ist aus der Gruppe bestehend aus, aber nicht beschränkt auf, hereditärer Teleangiektasie (HHT), Lungenembolie, Hirnembolie, COVID-19-Infektion und kardiovaskulärer Erkrankung.

6. Lösliches Endoglin zur Verwendung nach Ansprüchen 4, wobei die thrombotische Störung ein Schlaganfall ist.

7. Lösliches Endoglin zur Verwendung nach Ansprüchen 1, wobei der Thrombus durch Platzierung eines Zentralvenenkatheters, Restenose von Stents oder der Platzierung von Herzklappenprothesen verursacht wird.

8. Lösliches Endoglin zur Verwendung nach einem von Anspruch 1 bis 7, wobei das lösliche Endoglin in Kombination mit klassischer Behandlung von thrombotischen Störungen verwendet wird.

9. Lösliches Endoglin zur Verwendung nach einem von Anspruch 1 bis 8, wobei das lösliche Endoglin dem Subjekt in der Form einer pharmazeutischen Zusammensetzung verabreicht wird.

## Revendications

1. Endogline soluble pour une utilisation pour réduire ou prévenir la formation de thrombus chez un sujet qui en a besoin.

2. Endogline soluble pour une utilisation selon la revendication 1, dans laquelle le thrombus est causé par des troubles thrombotiques.

3. Endogline soluble pour une utilisation selon la revendication 1 ou 2, dans laquelle l'endogline soluble comprend ou fait référence à la séquence d'acides aminés de SEQ ID NO : 1 ou SEQ ID NO : 2.

4. Endogline soluble pour une utilisation selon les revendications 2 ou 3, dans laquelle le trouble thrombotique est choisi dans le groupe consistant en la télangiectasie hémorragique héréditaire, une mutation du gène de la prothrombine, des déficits en protéines naturelles empêchant la coagulation, des déficits en antithrombine, des déficits en protéines C, des déficits en protéines S, un taux élevé de facteur de coagulation, un système fibrinolytique anormal, l'hypoplasminogénémie, la dysplasminogénémie, une élévation des taux d'inhibiteur de l'activateur du plasminogène (PAI-1), la dysfibrinolyse, l'obésité, l'hypercoagulabilité gravidique, le syndrome des anticorps antiphospholipides, le cancer, l'homocystinémie, le syndrome des plaquettes collantes, l'embolie pulmonaire (EP), les troubles myéloprolifératifs, la polycythémie vraie ou la thrombocytose essentielle, hémoglobinurie paroxystique nocturne (HPN), les troubles thromboemboliques iatrogènes, la thrombocytopénie induite par l'héparine (HIT), la thromboembolie induite par le traitement de l'hémophilie, le syndrome inflammatoire de l'intestin, la colite ulcéreuse, la maladie de Crohn, le syndrome d'immunodéficience acquise (SIDA), la maladie à coronavirus, le syndrome néphrotique, la thrombose, la thrombose artérielle, la microthrombose aiguë, la thrombose microvasculaire distale, la thrombose veineuse profonde (TVP), la maladie de Paget-Schroetter, le syndrome de Budd-Chiari, la thrombose de la veine porte, la thrombose de la veine rénale, la thrombose des sinus veineux cérébraux, la thrombose de la veine jugulaire, la thrombose du sinus caverneux, l'ischémie cérébrale, la septicémie, l'anémie, la drépanocytose, le paludisme cérébral, l'embolie, l'embolie pulmonaire, l'embolie cérébrale, les maladies cardiovasculaires, l'ischémie cérébrovasculaire, l'infarctus cérébral aigu, l'accident vasculaire cérébral (AVC), l'accident vasculaire cérébral ischémique, l'accident vasculaire cérébral hémorragique, l'anévrisme, le trouble cognitif léger (MCI), les accidents ischémiques transitoires (AIT), l'infarctus du myocarde (ou crise cardiaque), la fibrillation auriculaire, la maladie coronarienne et l'insuffisance cardiaque congestive.

5. Endogline soluble pour une utilisation selon la revendication 4, dans laquelle les troubles thrombotiques sont sélectionnés dans le groupe consistant, de manière non exhaustive, en la télangiectasie hémorragique héréditaire (HHT), l'embolie pulmonaire, l'embolie cérébrale, l'infection par le COVID-19 et les maladies cardiovasculaires.

6. Endogline soluble pour une utilisation selon la revendication 4, dans laquelle les troubles thrombotiques sont des accidents vasculaires cérébraux.

7. Endogline soluble pour une utilisation selon la revendication 1, dans laquelle le thrombus est causé par la mise en place d'un cathéter veineux central, une resténose due à des stents ou à la mise en place de valves cardiaques prothétiques.

8. Endogline soluble pour une utilisation selon l'une quelconque des revendications 1 à 7, dans laquelle l'endogline soluble est utilisée en association avec le traitement classique des troubles thrombotiques.

9. Endogline soluble pour une utilisation selon l'une quelconque des revendications 1 à 8, dans laquelle l'endogline soluble est administrée au sujet sous la forme d'une composition pharmaceutique.
